# EUROPÄISCHE PATENTANMELDUNG

(11) **EP 3 587 390 A1**
(43) Veröffentlichungstag der Anmeldung: **01.01.2020**
(21) Anmeldenummer: 18179702.8
(22) Anmeldetag: 26.06.2018
(51) Int. Cl.: C07C 67/39, C07C 69/54, C07C 67/08, C07C 67/327

(54) **VERFAHREN ZUR HERSTELLUNG VON MMA IN HOHEN AUSBEUTEN**

(71) Anmelder: Röhm GmbH, 64293 Darmstadt (DE)
(72) Erfinder: KRILL, Steffen, 64367 Mühltal (DE); ZSCHUNKE, Florian, 60433 Frankfurt (DE); AIT AISSA, Belaid, 64287 Darmstadt (DE); TRESKOW, Marcel, 64293 Darmstadt (DE)
(74) Vertreter: Röhm Patent Association

(57) **Zusammenfassung**

Die vorliegende Erfindung betrifft ein Verfahren zur Herstellung von Methylmethacrylat durch direkte oxidative Veresterung von Methacrolein mit gegenüber dem Stand der Technik erhöhten Ausbeuten. Methylmethacrylat wird in großen Mengen zur Herstellung von Polymeren und Copolymeren mit anderen polymerisierbaren Verbindungen eingesetzt. Weiterhin ist Methylmethacrylat ein wichtiger Baustein für diverse, auf Methacrylsäure (MAS) basierenden Spezialester, die durch Umesterung mit dem entsprechenden Alkohol hergestellt werden können. Hieraus ergibt sich ein großes Interesse an möglichst einfachen, wirtschaftlichen und umweltschonenden Herstellungsverfahren für diesen Ausgangsstoff.

Insbesondere betrifft die vorliegende Erfindung eine optimierte Aufarbeitung des Reaktoraustrags der oxidativen Veresterung von Methacrolein, mittels der spezifische Nebenprodukte isoliert und anschließend zusätzlich zu Alkylmethacrylaten, insbesondere zu MMA umgesetzt werden können.

## Beschreibung

### Gebiet der Erfindung

Die vorliegende Erfindung betrifft ein Verfahren zur Herstellung von Methylmethacrylat durch direkte oxidative Veresterung von Methacrolein mit gegenüber dem Stand der Technik erhöhten Ausbeuten. Methylmethacrylat wird in großen Mengen zur Herstellung von Polymeren und Copolymeren mit anderen polymerisierbaren Verbindungen eingesetzt. Weiterhin ist Methylmethacrylat ein wichtiger Baustein für diverse, auf Methacrylsäure (MAS) basierenden Spezialester, die durch Umesterung mit dem entsprechenden Alkohol hergestellt werden können. Hieraus ergibt sich ein großes Interesse an möglichst einfachen, wirtschaftlichen und umweltschonenden Herstellungsverfahren für diesen Ausgangsstoff.

Insbesondere betrifft die vorliegende Erfindung eine optimierte Aufarbeitung des Reaktoraustrags der oxidativen Veresterung von Methacrolein, mittels der spezifische Nebenprodukte isoliert und anschließend zusätzlich zu Alkylmethacrylaten, insbesondere zu MMA umgesetzt werden können.

### Stand der Technik

Methylmethacrylat (MMA) wird heute mittels diverser Verfahren, die von C₂- C₃- oder C₄-Bausteinen ausgehen, hergestellt. In einem dieser Verfahren wird MMA durch Oxidation von Isobutylen oder *tert*-Butanol mit Luftsauerstoff in der Gasphase an einem heterogenen Kontakt zu Methacrolein (MAL) und anschließender oxidativer Veresterungsreaktion von Methacrolein unter Verwendung von Methanol erhalten. Dieses von ASAHI entwickelte Verfahren ist unter anderem in den Druckschriften US 5,969,178 und US 7,012,039 beschrieben. Nachteil dieses Verfahrens ist insbesondere ein sehr hoher Energiebedarf.

In folgendem Schema 1 ist die Herstellung von MMA nach dem sogenannten Asahi Verfahren ausgehend von C4 (Isobuten oder *tert*-Butanol), mit Zwischenisolierung von MAL und anschließender oxidativer Veresterung (abgekürzt "DOE") des MAL mit Methanol zu MMA, unter Bildung von Methacrylsäure (MAS) als Nebenprodukt, schematisch abgebildet.

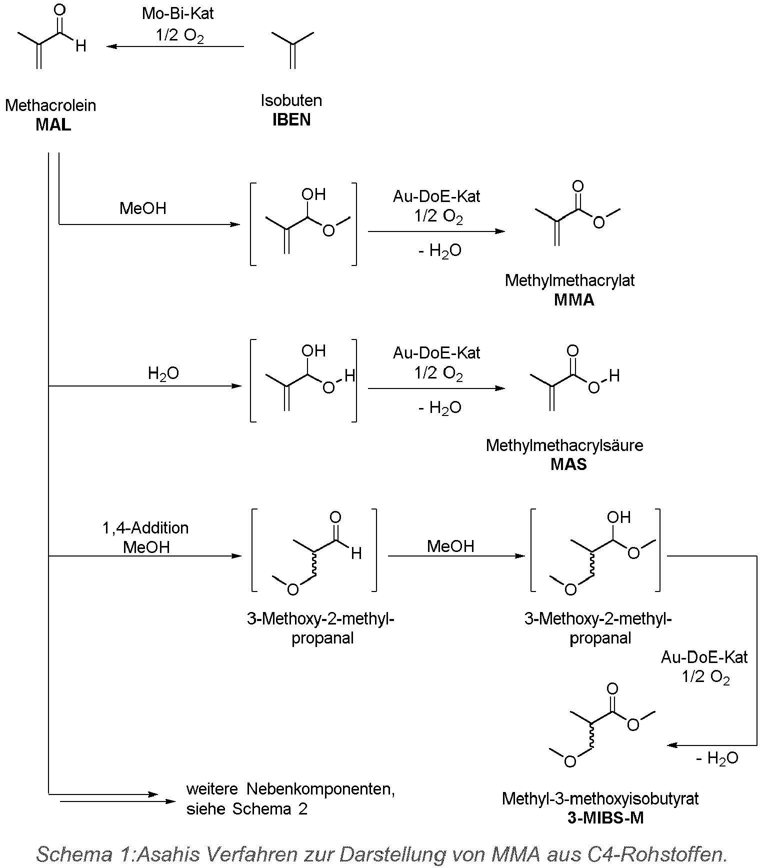

In einer Weiterentwicklung des Verfahrens wird das Methacrolein in der ersten Stufe aus Propanal und Formalin gewonnen. Ein solches Verfahren ist in WO 2014/170223 beschrieben.

In US 5,969,178 ist ein solches Verfahren zur oxidativen Umsetzung von Isobuten oder *tert*-Butanol zu Methacrolein und die folgende oxidative Veresterung zu MMA beschrieben. In dieser zweiten Stufe wird eine flüssige, im Wassergehalt reduzierte Mischung aus Methacrolein und Methanol mit molekularem Sauerstoff und einem Palladiumkatalysator umgesetzt, wobei dieser zumeist auf einem Träger als Palladium- Blei-Katalysator vorliegt. Aus dem Rohprodukt der oxidativen Veresterung wird darauf in einer ersten Destillationsstufe ein Gemisch aus Methacrolein und Methanol unterhalb des Kopfes der Kolonne abgetrennt, während über Kopf leichtsiedende Bestandteile entfernt werden. Der MMA-haltige Sumpf wird darauf in eine zweite Destillationsstufe geführt, in der über Kopf ein Azeotrop aus Methanol und gesättigten Kohlenwasserstoffen abgetrennt wird. Der Sumpf, enthaltend das Roh-MMA wird einer weiteren Aufarbeitung zugeführt, während aus der über Kopf gewonnen Fraktion mittels eines Phasentrenners und einer dritten Destillationskolonne Methanol isoliert und zurück in den Reaktor geführt wird. Dabei ist zu berücksichtigen, dass das Methanol aufgrund des gebildeten Azeotrops relativ viel Wasser enthalten kann und damit einer Entwässerung zugeführt werden muss.
Als Alternative zu diesem Verfahren offenbart die US 5,969,178 die Aufarbeitung in nur einer Kolonne, wobei sich bei dieser der Zulauf zwingend oberhalb des Sumpfes befindet. Über Kopf werden dieser Kolonne leichtsiedende Bestandteile aus dem Reaktoraustrag entfernt. Im Sumpf verbleibt eine Mischung aus Roh-MMA und Wasser, welche einer weiteren Aufarbeitung zuzuführen ist. Über einen Seitenstrom, dessen genaue Lage zunächst bestimmt werden muss und die durch Hinzufügen verschiedener Siedeböden eingestellt werden kann, wird der Kolonne schließlich eine Mischung aus Methacrolein und Methanol, gedacht zur Zurückführung in den Reaktor, entnommen. US 5,969,178 weist dabei selbst darauf hin, dass ein solches Verfahren aufgrund diverser Azeotrope schwierig durchzuführen ist. Darüber hinaus spielt dabei insbesondere Methacrylsäure, die immer als Nebenprodukt vorliegt eine große Rolle. Nach diesem Verfahren, auch wenn die US 5,969,178 darüber schweigt, würde die Methacrylsäure derart abgetrennt werden, dass sie in einer der Entsorgung zuzuführenden Phase verbleibt und sich eine Isolierung nur bedingt lohnen würde. Damit sinkt jedoch die Gesamtausbeute an methacrylischen Produkten dieses Verfahrens.

In US 7,012,039 wird eine etwas abweichende Aufarbeitung des Reaktoraustrags der oxidativen Veresterung offenbart. Hier wird in einer ersten Destillationsstufe über Siebböden Methacrolein über Kopf abdestilliert und die wässrige, MMA haltige Mischung aus dem Sumpf in einen Phasenseparator geleitet. In diesem wird die Mischung durch Zugabe von Schwefelsäure auf einen pH-Wert von ca. 2 eingestellt. Die Trennung des schwefelsauren Wassers von der organischen bzw. ÖI-Phase erfolgt dann mittels Zentrifugieren. Diese organische Phase wird in einer weiteren Destillation in hochsiedende Bestandteile und eine MMA haltige Phase, welche über Kopf abgezogen wird, getrennt. Die MMA haltige Phase wird danach in einer dritten Destillation von niedrigsiedenden Bestandteilen getrennt. Darauf folgt noch eine vierte Destillation zur endgültigen Reinigung. Problematisch an diesem Verfahren ist die Schwefelsäure, die in großen Mengen zugegeben werden muss und in Teilen der Anlage korrosiv wirken kann. Entsprechend müssen diese Teile, wie insbesondere der Phasenseparator oder auch die zweite Destillationskolonne aus dafür geeigneten Materialien gefertigt sein. Weiterhin schweigt auch die US 7,012,039 über den Umgang mit der gleichsam anfallenden Methacrylsäure oder dem im Produkt verbleibenden restlichen Methanol. Es ist jedoch zu vermuten, dass die erstgenannte in den Destillationsstufen mit abgetrennt wird, während das Methanol nur teilweise mit dem Methacrolein gewonnen und zurückgeführt werden kann, während der Rest wahrscheinlich in der dritten Destillationsstufe verloren geht.

WO 2014/170223 beschreibt ein ähnliches Verfahren wie US 7,012,039. Es besteht nur der Unterschied, dass in der eigentlichen Reaktion mittels Zugabe einer methanolischen Natriumhydroxidlösung in einer Kreislaufführung der pH-Wert eingestellt wird. Führt man das Verfahren ohne pH Regulierung durch, wie es in den beiden genannten Publikationen beschrieben ist, kommt es zu einer "Versauerung" der Reaktion. In einem pH Bereich unterhalb 7 führt dies zur vermehrten Bildung des Acetals des Methacroleins, welches aufwendig abgetrennt bzw. hydrolytisch gespalten werden müsste. Zudem ist die Aktivität des Oxidationskatalysators unter anderem vom pH Wert abhängig. Bei Bedingungen unterhalb pH 7 zeigt der Katalysator mit sinkendem pH Wert der Reaktionsmatrize zunehmend geringere Aktivität, was nicht wünschenswert ist. Die pH Regulierung dient zudem unter anderem dazu, den Katalysator zu schonen. Weiterhin ist die Abtrennung der wässrigen Phase in der Phasenseparation aufgrund des Salzgehalts einfacher. Allerdings führt dies auch dazu, dass die entstehende Methacrylsäure als Natriumsalz vorliegt und später mit der wässrigen Phase abgetrennt und verworfen wird. Bei der Variante einer Schwefelsäurezugabe in der Phasenseparation wird die freie Säure zwar wiedergewonnen, aber auf Kosten von anfallendem Natrium(hydrogen)sulfat, welches bei der Entsorgung zu anderen Problemen führen kann.

Grundsätzlich und in Zusammenfassung des Standes der Technik fallen bei der oxidativen Veresterung von Methacrolein bzw. auch Acrolein, in Gegenwart von Methanol als Alkohol allgemein bei der direkten oxidativen Veresterung ungesättigter Aldehyde, verschiedene schwersiedende Komponenten an. Diese Schwersieder sieden gegenüber dem als Produkt gewünschten Methylmethacrylat (MMA) höher und müssen bei der späteren Isolierung des MMAs zur Erzeugung einer geeigneten Reinheit des Monomers von deutlich größer als 99 Gew% somit von MMA abgetrennt werden. Diese Nebenprodukte, insbesondere Methacrylsäure, Methoxyisobuttersäuremethylester, dimeres Methacrolein (ein Aldehyd) und der korrespondierende Ester des dimeren Methacrolein entstehen in signifikanten Mengen und können die Ausbeute an gewünschtem MMA deutlich negativ beeinflussen. Da sie von entstehendem MMA abgetrennt werden müssen, besteht ebenfalls die Notwendigkeit diese Stoffe einer Entsorgung zuzuführen, im einfachsten Fall einer Verbrennung, einer thermischen Verwertung unter Dampfgewinnung bzw. einem biologischen Abbau in einer Kläranlage.

Eines der Nebenprodukte ist Methacrylsäure, die sich in der DOE Reaktion in Gegenwart von Wasser bildet, was üblicherweise bei der kontinuierlichen Durchführung der Reaktion in einer stationären Konzentration zwischen 2 und 20 Gew% im Reaktor anwesend ist. Führt man die DOE Reaktion bei konstantem pH durch, so wird diese sich bildende Methacrylsäure zumindest anteilig mit alkalischen bzw. basischen Hilfsstoffen, im einfachsten Fall Alkaliverbindungen neutralisiert.
In der Reaktion bildet sich ebenfalls Methoxyisobutyral, das Michael-Additionsprodukt von Methanol an Methacrolein. Dieses Methoxyisobutyral wird unter den Bedingungen der DOE in Gegenwart eines sauerstoffhaltigen Gases und z.B. Methanol zumindest teilweise zum Methoxyisobuttersäuremethylester (MIBS-M) als Nebenreaktion umgesetzt. Die Reaktion ist alkalisch katalysiert, wird also durch die Zugabe einer Base die man einsetzt um den pH der DOE Reaktion zu kontrollieren zwangsläufig gebildet. Allgemein wird beobachtet, dass die Bildung von Methoxyisobutyral und damit die konsekutive Bildung von MIBSM mit steigendem pH ausgeprägter sind. Je nach eingesetztem Katalysator und je nach gewähltem, stationären pH der DOE Reaktion im Reaktor bilden sich zwischen 0,1 und bis zu 5% dieses Nebenproduktes.

Im Stand der Technik zum Beispiel in der deutschen Auslageschrift 1279015, Knörr et al. ist beschrieben, wie Alkoxypropionsäurealkylester thermisch und katalytisch gespalten werden können, wobei ungesättigte Acrylsäureester erhalten werden. Hierbei werden Reinstoffe als Edukte verwendet und es wird eine reine ß-Eliminierung beschrieben.

In der WO2016166525A1 Anmeldung beschreibt Lucite die katalytische, baseninduzierte Spaltung von Methoxyisobuttersäuremethylester in Methanol und Methylmethacrylat. Hierbei werden Reinstoffe als Edukte unter Schutzgasatmosphäre bei 95 °C eingesetzt. Der Umsatz liegt jedoch nur bei niedrigen 37%.

Im folgenden Schema 2 ist die Reaktionmatrix (exemplarisch mit Ethylen und Syngas und Formalin zum Methacrolein; wie beschrieben ist auch der Methacrolein Zugang ausgehend von Isobuten oder *tert*-Butanol möglich) dargestellt, insbesondere die Bildung des Zielprodukts MMA sowie die schwersiedenden Nebenprodukte MAS, MIBSM, DIMAL und DIMAL Ester sind aufgeführt: 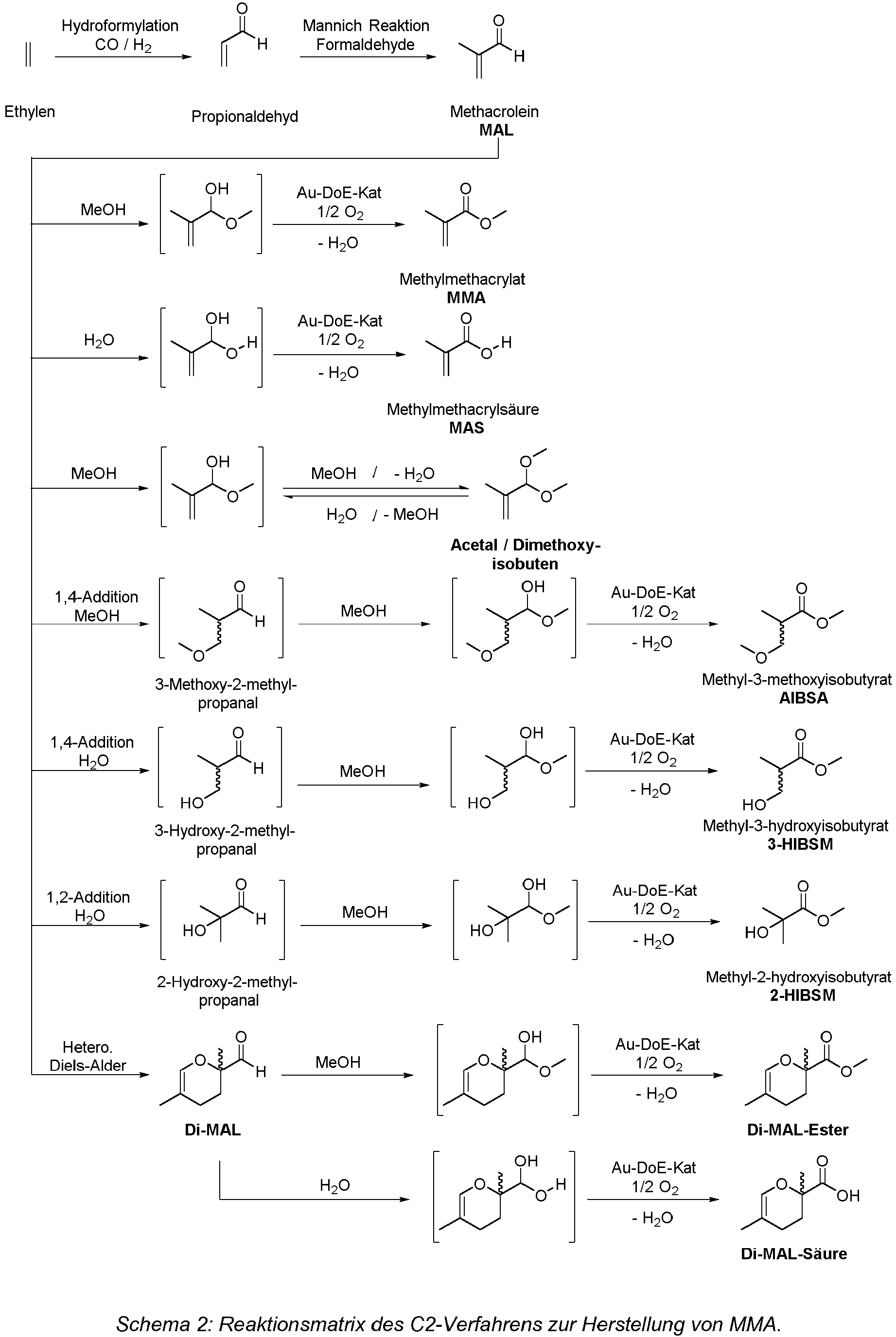 Es resultiert in der Produktmatrix der DOE Reaktion eine Mischung, enthaltend Wasser, Methanol, MMA, freie Methacrylsäure neben alkalisch neutralisierter Methacrylsäure, beispielsweise Natrium Methacrylat, sowie den weiteren in Schema 2 beschriebenen Nebenprodukten.

Zusammengefasst sind die folgenden Aspekte der Verfahren gemäß Stand der Technik vor allem in der Kombination miteinander verbesserungswürdig:
- Möglichst hohe Ausbeute an MMA
- Umwandlung des Nebenprodukts Methacrylsäure zum Methylmethacrylat und Isolierung
- Umwandlung des Nebenprodukts Methoxyisobuttersäuremethylester (MIBSM) zu Methanol und MMA sowie Rückführung des entstehenden Methanols bzw. die Verwendung des MIBSM im Sinne einer Kreuzumesterung zur Umwandlung von MAS in MMA
- zumindest anteilige Umwandung von dimerem Methacrolein und dem entsprechenden DiMAL Ester in MMA und Methacrolein
- Möglichst hoher Grad des Recyclings von Nebenprodukten
- Möglichst saubere Entsorgungsströme bzw. Abgase

### Aufgabe

In Anbetracht des Standes der Technik war es daher Aufgabe der vorliegenden Erfindung, ein technisch verbessertes Verfahren zur oxidativen Veresterung von Methacrolein zur Verfügung zu stellen, das nicht mit den Nachteilen herkömmlicher Verfahren behaftet ist und zu gegenüber dem Stand der Technik höheren Ausbeuten führt.

Insbesondere war es Aufgabe der vorliegenden Erfindung, eine Verbesserung der Aufarbeitung des Rohproduktes einer oxidativen Veresterung von Methacrolein mit Methanol zu MMA bereitzustellen und damit die Gesamtausbeute eines solchen Prozesses gegenüber dem Stand der Technik zu verbessern.

Darüber hinaus war es Aufgabe, möglichst viele im Prozess gebildete Nebenprodukte, insbesondere Methoxyisobuttersäurealkylester und Methacrylsäure, in möglichst hohem Maße zu isolieren und ausbeutesteigernd für die Herstellung von Alkylmethacrylaten zur Verfügung zu stellen. Eine weitere explizite Aufgabe der Erfindung bestand darin, einen Prozess zu finden, der es möglichst erlaubt, mehrere dieser Nebenprodukte in einem einzigen Prozessschritt zu MMA umsetzen, und bevorzugt bei der Aufarbeitung des Rohproduktes auf Verfahrensschritte aufbaut, die eh für die Isolierung von MMA benötigt werden. Insbesondere bezieht sich dies auf eine Lösung der Aufgaben, die auf bereits aus anderen Gründen vorliegenden Kolonnen, Phasentrennern, Extraktoren oder allgemeinem Equipment beruhen.

Insbesondere bestand auch die Aufgabe, ein Verfahren zur Verfügung zu stellen, das mit einem möglichst geringen Entsorgungsaufwand, insbesondere durch ein reduziertes Anfallen organischer Bestandteile und Säuren im Abfallstrom, betrieben werden kann.

### Lösung

Gelöst werden die Aufgaben durch ein Verfahren zur Herstellung von Alkylmethacrylaten , bei dem in einer ersten Reaktionsstufe in einem Reaktor I Methacrolein hergestellt und dieses in einer zweiten Reaktionsstufe in einem Reaktor II oxidativ mit einem Alkohol, bevorzugt mit Methanol zu einem Alkylmethacrylat, bevorzugt entsprechend zu MMA verestert wird, wobei sich das Verfahren erfindungsgemäß dadurch auszeichnet, dass a. der Reaktoraustrag des Reaktor II in eine den überwiegenden Teil des Alkylmethacrylat enthaltene Fraktion und eine zweite Fraktion, enthaltend Methacrylsäure und einen Alkoxyisobuttersäurealkylester (AIBSM) getrennt wird. Im bevorzugten Falle, dass es sich bei dem Alkohol um Methanol handelt, handelt es sich bei dem MIBSM um Methoxyisobuttersäuremethylester (MIBS-M).

Weiterhin ist das erfindungsgemäße Verfahren dadurch gekennzeichnet, dass b. diese zweite Fraktion in einem Reaktor III derart umgesetzt wird, dass aus dem MIBSM und der Methacrylsäure weiteres Alkylmethacrylat gebildet wird. Die chemischen Umwandlungen sind im Schema 3 dargestellt, wobei exemplarisch die Umsetzung an Hand von MAS und MIBS-M gezeigt ist:

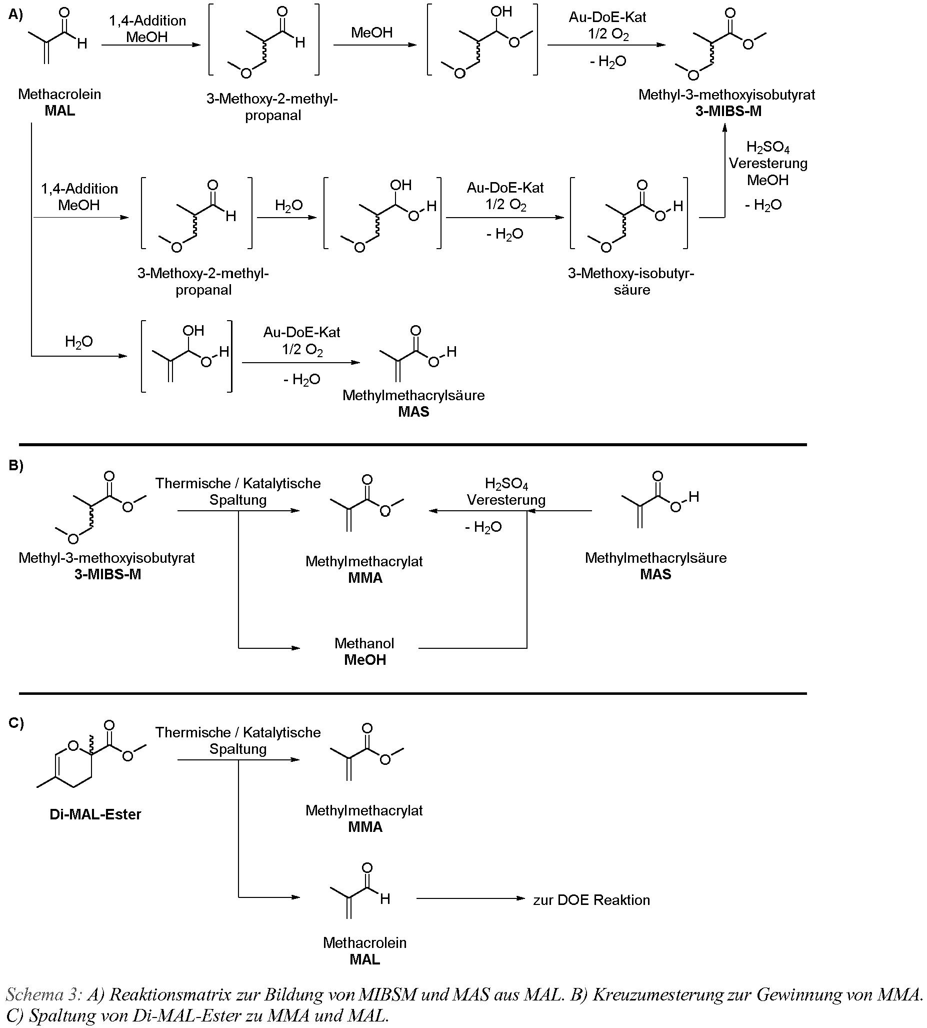

Sehr vorteilhaft kann hier gezeigt werden, dass bei der Spaltung des einen Nebenprodukts (MIBS-M) Methanol freigesetzt wird, was im Sinne einer Kreuzumesterung zur Umsetzung des zweiten Nebenprodukts, nämlich Methacrylsäure MAS, benötigt wird, um MMA zu bilden. Auch die in Spuren gebildete 3-Methoxyisobuttersäure wird bei dieser Umsetzung zunächst zu 3-MIBS-M und dann zu MMA umgesetzt.
Alle diese Reaktionen laufen synchron ab, bevorzugt in einer einzigen Vorrichtung oder einem einzelnen Reaktor, so dass letztlich eine Vielzahl von Nebenprodukten zum Zielprodukt MMA umgesetzt werden.

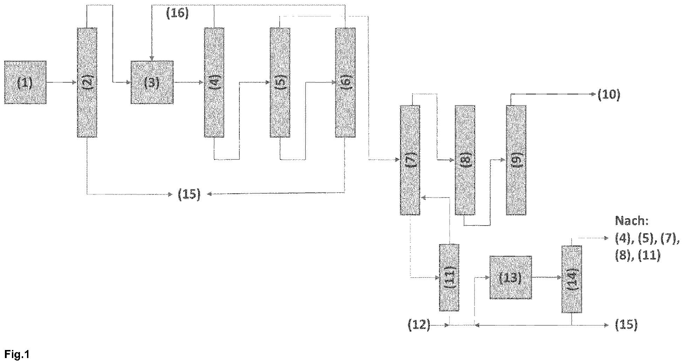

Das Verfahren zur Synthese von MMA, welches die beiden oben aufgeführten Reaktionsstufen aufweist, kann insbesondere in US 5,969,178, US 7,012,039 und WO 2014/170223 nachgelesen werden. Ein mögliches, erfindungsgemäßes Fließbild ist dabei in Fig. 1 aufgeführt.
Dabei ist erfindungsgemäß die erste Stufe des Verfahrens zur Synthese des Methacroleins frei wählbar. Das erfindungsgemäße Verfahren ist sowohl auf eine Erststufen-Synthese auf Basis von *tert*-Butanol oder Isobutylen, als auch auf Basis von Propanal und Formalin anwendbar.
Auch bei der Herstellung von Methacrolein auf Basis von Propanal und Formalin kommen im Prinzip zwei Verfahrensvarianten in Frage, die erfindungsgemäß zu einer Qualität des Methacroleins führen, die in der DOE Reaktion eingesetzt werden kann. Zum einen können Propanal und Formalin in einem gerührten oder umgepumpten Reaktor bei Temperaturen von 20°C bis 120°C bei Drücken von 1 bar bis 10 bar umgesetzt werden. Dabei werden Reaktionszeiten größer 10 min benötigt, um ausreichende Umsätze zu erzielen. Zum anderen kann man aus diesen Edukten MAL herstellen, wobei man bei mittleren Druck zwischen 10 und 100 bar bei höheren Temperaturen zwischen 120°C und 250°C die Reaktion mit einer Reaktionszeit von 2 Sekunden bis 20 Sekunden zu gewünschten, hohen Ausbeuten kommt.

Bevorzugt wird die oxidative Veresterung in flüssiger Phase bei einem Druck von 2 bis 100 bar, bevorzugt bei einem Druck im Bereich von 2 bis 50 bar und einer Temperatur im Bereich von 10 bis 200 °C mit einem heterogenen Katalysator durchgeführt. Bei dem heterogenen Katalysator handelt es sich in der Regel um geträgerte, goldhaltige Nanopartikel mit einer Teilchengröße kleiner 20 nm, bevorzugt zwischen 0,2 und 20 nm. Die Reaktionsstufe (A) kann eine optionale und weniger bevorzugte Destillationskolonne II zur Abtrennung von Leichtsiedern, wie verbliebenen Propanal, und/oder von Schwersiedern, wie dimeres Methacrolein, aufweisen.

Besonders bevorzugt erfolgt in Schritt a. die Trennung mittels mindestens einer Extraktion und/oder einer Destillation. Dabei können auch mehrere Destillationsschritte oder Extraktionsschritte, wie auch Kombinationen aus mindestens einer Destillation und mindestens einer Extraktion eingesetzt werden.

Es hat sich als ganz besonders bevorzugt in dem erfindungsgemäßen Verfahren erwiesen, das Verfahren derart durchzuführen, dass der Reaktoraustrag des Reaktor II in einer ersten Destillationskolonne von Methacrolein und teilweise von dem Alkohol befreit wird, wobei ein Strom, enthaltend Alkylmethacrylat, Wasser, ein Alkalimethacrylat und/oder Methacrylsäure, MIBSM und Alkohol, erhalten wird. Anschließend wird dieser Strom mit einer starken Säure versetzt und in einer Extraktion in eine hydrophobe Phase, enthaltend Alkylmethacrylat, MAS und MIBSM und eine hydrophile Phase, enthaltend Wasser, den Alkohol, und verbleibende geringere Mengen an Haupt und Nebenprodukten der Reaktion.

Alternativ dazu und genauso bevorzugt, wird das erfindungsgemäße Verfahren derart ausgeführt, dass der Reaktoraustrag des Reaktor II in einer ersten Destillationskolonne von Methacrolein und teilweise von dem Alkohol befreit wird, wobei ein Strom, enthaltend Alkylmethacrylat, Wasser, ein Alkalimethacrylat und/oder Methacrylsäure, MIBSM und Alkohol, erhalten wird. Die Methacrylsäure kann an dieser Stelle im Verfahren in Form der freien, organischen Säure bzw. als Alkalisalz bzw. in Form einer Mischung von freier Säure und Alkalisalz vorliegen. Optional wird durch Ansäuerung, wie beispielsweise ein Versetzen des Gemisches mit einer Brönstedtsäure, die als Salz vorliegende Säure in die freie Säure überführt.
Dieser Strom wird anschließend einer Extraktion unterzogen, wobei eine organische Phase resultiert, die hauptsächlich MMA enthält, aber auch anteilig die organischen Nebenprodukte MAS und MIBSM. Die nach Extraktion resultierende organische Phase wird in einer zweiten Destillation in eine leichtsiedende Phase, enthaltend Alkylmethacrylat und den Alkohol und eine schwersiedende Phase, enthaltend Wasser, MIBSM und Methacrylsäure, getrennt.

Somit ist in dem erfindungsgemäßen Verfahren eine Abtrennung des Wertstoffes MMA von den Nebenprodukten der DOE Reaktion, insbesondere in Reaktor II, besonders vorteilhaft. Insbesondere eine Abtrennung des MMA von MAS, MIBSM, DIMAL und DIMAL-Ester sowie den Isomeren des HIBS kann so erfindungsgemäß überraschend realisiert werden.
Diese oben beschriebene schwersiedende Phase wird darauf gemäß dem zweiten erfindungsgemäßen Aspekt einer weiteren Reaktion unterzogen, wobei die Nebenprodukte der DOE zum gewünschten Hauptprodukt MMA umgesetzt werden. Das zusätzlich in Reaktor III gebildete MMA erhöht die Gesamtausbeute des Verfahrens signifikant. Vorteilhafterweise wird das in Reaktor III erhaltene MMA Rohprodukt einer oder mehreren Aufarbeitungskolonnen des Hauptverfahrens zugeführt.

Die Umsetzung in Reaktor III erfolgt wiederum bevorzugt bei einer Temperatur von mindestens 90 °C, besonders bevorzugt von mindestens 110 °C, ganz besonders bevorzugt zwischen 120°C und 170 °C. So kann die Umsetzung rein thermisch ohne Zugabe eines Katalysators durchgeführt werden. Besonders bevorzugt erfolgt die Umsetzung thermisch in Gegenwart eines Katalysators, bei dem es sich insbesondere um eine Brönstedtsäure handeln kann.
In einer dritten Alternative kann die Umsetzung auch in Gegenwart eines solchen Katalysators bei Temperaturen unterhalb von 130 °C erfolgen.

Besonders bevorzugt ist es, wenn es sich bei der Brönstedtsäure um eine starke Säure handelt. Erfindungsgemäß wird unter einer starken Säure eine derartige Säure verstanden, die stärker ist als Methacrylsäure. Das bedeutet, dass diese Säure unter Normalbedingungen einen geringeren pKs-Wert als Methacrylsäure aufweist. Eine insbesondere bevorzugte anorganische Säure ist dabei Schwefelsäure. Bei den weniger bevorzugten organischen Säuren kann es sich beispielsweise um Methansulfonsäure oder Toluolsulfonsäure handeln. Beispiel für eine weitere geeignete mineralische Säure ist Phosphorsäure. Schwefelsäure hat sich dabei als besonders geeigneter Katalysator erwiesen. Allgemein ist es vorteilhaft eine Katalysatorsäure in Reaktor III zu verwenden, die identisch ist, mit der Säure, die man zum Ansäuern und Freisetzen der MAS aus der Alkali Methacrylsäure einsetzt. Eine dritte Funktion der Säure in diesem Verfahren ist die Zersetzung von störenden Acetalen des Methacroleins. Somit ist man in der Lage im besten Fall mit nur einer einzigen Säure innerhalb des Anlagenverbunds auszukommen. Ein besonders geeignetes Beispiel einer solchen universal einsetzbaren Säure ist Schwefelsäure, die je nach Einsatzfunktion verschieden stark konzentriert ist bzw. eingestellt wird.

Es hat sich im Weiteren als bevorzugt erwiesen, den Reaktoraustrag des Reaktors III mit der nach Abtrennung des MIBSM und der Methacrylsäure erhaltenen, Alkylmethacrylat-haltigen Fraktion zur weiteren Aufarbeitung zusammenzuführen. Diese zweite Fraktion kann dabei zum einen der nach der erfindungsgemäßen Trennung in Verfahrensschritt a), nicht der MIBSM enthaltene Strom sein. Es ist jedoch günstiger, diesen Strom zunächst in einem oder mehreren Schritten zunächst vor zu reinigen, bevor beide Fraktionen zur weiteren Reinigung zusammengeführt werden. Die Wahl der Vorreinigung hängt beispielsweise von dem für den ersten Verfahrensschritt in Reaktor I gewählten Prozess und den darin eingesetzten Rohstoffen ab. Bei dieser Vorreinigung des eigentlichen Roh-Alkylmethacrylats kann es sich beispielsweise um eine Hochsieder-, eine Niedrigsiederkolonne oder um beide in Reihe geschalteten Destillationen handeln. Alternativ oder parallel dazu ist es natürlich auch möglich, erst den Reaktoraustrag aus Reaktor III vor zu reinigen, bevor diese Fraktion mit der zuvor genannten anderen Fraktion zusammengeführt wird. Auch bei dieser Reinigung kann es sich um eine oder mehrere Extraktionen bzw. Destillationen oder Kombinationen daraus handeln. Die aus der Aufreinigung erhaltene, eingesetzte Säure kann dazu optional wieder in den Reaktor III recycliert werden. Besonders bevorzugt befindet sich direkt hinter Reaktor II eine Destillationskolonne zur Abtrennung von MAL. Dieses kann dann in Reaktor II oder einen vorgeschalteten Reinigungsschritt zurückgeführt werden.

Verfahrensgemäß ist als Teilaspekt der vorliegenden Erfindung die Herstellung von Methacrolein im beschriebenen Reaktor I. Einen guten Überblick über die Methoden und Verfahren zur Herstellung von Methacrolein gibt Ullmanns Encyclopedia of industrial chemistry, 2012, Wiley-VCH Verlag GmbH, Weinheim, DOI: 10.1002/14356007.a01_149.pub2.
Wir konnten zeigen, dass mehrere Verfahrensvarianten prinzipiell geeignet sind, um Methacrolein herzustellen, die exemplarisch wie folgt aussehen können:
a. Herstellung von Methacrolein aus Propanal und Formalin in Gegenwart von Katalysatoren, bevorzugt homogenen Säuren, mineralisch oder organisch, und organischen Aminen unter erhöhtem, absolutem Druck von größer 2 bar. Beispiele sind unter anderem in EP 2 998 284 A1 oder in US 7,141,702, JP 3069420, JP 4173757, EP 0 317 909 oder US 2,848,499 beschrieben. Auch geeignet ist das Verfahren gemäß DE 32113681, wobei hohe Umsätze und Ausbeuten bei Verweilzeiten im Sekundenbereich erzielt werden. Bei letzter Publikation wird aber auch auf die Erzeugung von dimerem Methacrolein ("DIMAL") hingewiesen, was letztlich einen Verlust darstellt. Diese Verfahren verlaufen bei erhöhten Temperaturen und Drücken mit dem Vorteil einer niedrigen Verweilzeit der Reaktanden und somit vergleichsweise geringen Reaktorvolumina.
b. US 4,408,079 steht exemplarisch im Gegensatz zu den zuvor genannten Verfahren für Prozesse mit eher niedrigen Temperaturen und deutlich höheren Verweilzeiten, die somit höhere Reaktorvolumina benötigen. Diese Verfahren werden bei absoluten Drücken von über 2 bar durchgeführt. Gleichzeitig werden aber deutlich höhere Katalysatormengen - teilweise von sogar 50 mol% - benötigt, wobei aber die Katalysatorlösungen auch recycliert werden können. Üblicherweise werden bei diesen Verfahrensvarianten gerührte oder umgepumpte Rührkessel oder Kaskaden dieser Kessel verwendet. Kennzeichnend bei dieser Verfahrensvariante sind eher höhere DIMAL-Gehalte als Nebenprodukt, was auf längere Verweilzeiten und somit verstärkt vorkommende Diels-Alder-Reaktion von Methacrolein zurückzuführen ist.
c. Die dritte Verfahrensvariante zur Herstellung von Methacrolein ist dadurch gekennzeichnet, dass Isobuten oder *tert*-Butanol in der Gasphase an einem heterogenen Kontakt mit Wasserdampf und sauerstoffhaltigen Gasen bei Temperaturen von über 300°C umgesetzt und anschließend isoliert wird. Eine Vielzahl von Untervarianten und einsetzbarer Katalysatorsysteme sowie Isolierungsoptionen sind im einschlägigen Stand der Technik beschrieben. Eine gute Übersicht hierzu gibt folgende Referenz: Trends and Future of Monomer-MMA Technologies, K. Nagai & T. Ui, Sumitomo Chemical Co., Ltd.,Basic Chemicals Research Laboratory, 2005, http://ww.sumitomo chem.co.jp/english/rd/report/theses/docs/20040200_30a.pdf. werden Diese Verfahren mit C4 -Rohstoffen werden vor allem im asiatischen Raum großtechnisch durchgeführt.

Es ist bevorzugt, dass es sich bei der ersten Reaktionsstufe in Reaktor I um eine Umsetzung von Propanal mit Formalin handelt. In diesem Fall ist es dann weiterhin bevorzugt, dass der Reaktoraustrag des Reaktor II in einer ersten Destillationskolonne von Methacrolein und teilweise von dem Alkohol befreit wird, wobei ein Strom, enthaltend Alkylmethacrylat, Wasser, ein Alkalimethacrylat und/oder Methacrylsäure, MIBSM und Alkohol, erhalten wird. Dieser Strom wird dann anschließend in einer zweiten Destillation in eine leichte Phase, enthaltend Alkylmethacrylat und den Alkohol und eine schwere Phase, enthaltend Wasser, MIBSM, Methacrylsäure, dimeres Methacrolein und optional einen Alkylester des dimeren Methacroleins, getrennt.

Das dimere Methacrolein in Reaktor III in dieser Verfahrensvariante kann anschließend in Methacrolein gespalten werden. Dabei ist es dann auch möglich, den optional vorliegende Alkylester des dimeren Methacroleins in Methacrolein und das dem eingesetzten Alkohol entsprechende Alkylmethacrylat zu spalten. Das so jeweils gewonnene Methacrolein kann dann von dem Alkylmethacrylat in einer späteren Destillationsstufe getrennt und zurück in Reaktor II geführt werden.

Alternativ zu der beschriebenen Verfahrensvariante, bei der es sich bei der ersten Reaktionsstufe in Reaktor I um eine Umsetzung von Propanal mit Formalin handelt, kann es sich bei dieser ersten Reaktionsstufe auch um eine Oxidation von *tert*-Butanol und/oder Isobuten handeln.

In einer solchen Variante ist es dann besonders bevorzugt, den Reaktoraustrag des Reaktor II in einer ersten Destillationskolonne von Methacrolein und teilweise von dem Alkohol zu befreien. Dabei erhält man einen Strom, der Alkylmethacrylat, Wasser, ein Alkalimethacrylat und/oder Methacrylsäure, MIBSM und Alkohol enthält. Dieser Strom wird üblicherweise zunächst mit Säure behandelt, beispielsweise Mineralsäuren wie etwa Schwefelsäure, wobei der Hauptteil des Alkalimethacrylates neutralsiert und somit die freie Methacrylsäure gebildet wird.
Dieser Strom wird dann anschließend in einer zweiten Destillation und/oder einer Extraktion in eine leichte oder hydrophobe Phase, enthaltend Alkylmethacrylat und die Nebenprodukte der Umsetzung, nämlich den Großteil der gebildeten Methacrylsäure, MIBSM, sowie DIMAL, DIMAL-Ester und kleinere Mengen an Wasser und Methanol, und eine schwere, hydrophile Phase getrennt. Diese zweite, überwiegend wässrige Phase enthält naturgemäß wenig organische Produkte und besteht hauptsächlich aus Wasser und Methanol und enthält Alkali- oder Erdalkali Salz aus der Neutralisation.

Als Teilaspekt der vorliegenden Erfindung wird Methacrolein in Gegenwart eines sauerstoffhaltigen Gases bei moderaten Temperaturen zwischen 20 und 150 °C bei moderaten Drücken zwischen 1 und 20 bar in Gegenwart eines heterogenen sphärischen Edelmetall-haltigen Katalysators umgesetzt. Eine Vielzahl von Katalysatoren kann für diese oxidative Veresterung von MAL mit Methanol zu MMA eingesetzt werden:
Die erste bekannte Anwendung der direkten oxidativen Veresterung von MAL mit Methanol zu MMA wurde von Asahi mit einem Pd-Pb-Katalysator, der sich auf einem oxidischen Träger befindet, durchgeführt. In der US 6,040,472 werden diese Katalysatoren beschrieben, die jedoch im Vergleich mit nur unzureichenden Aktivitäten und Selektivitäten zu MMA führen. Es handelt sich in diesem Fall um Pd-Pb-haltige Katalysatoren mit Schalenstruktur. Die Selektivitäten zu MMA werden mit bis zu 91 % und die Raum-Zeit-Ausbeute mit bis zu 5,3 mol angegeben. Auch hier ist die Blei-Dotierung für die Ausbildung der aktiven Oxidationsspezies entscheidend, erzeugt aber durch schleichenden Verlust an Blei Ionen die oben beschriebenen Nachteile. Nebenprodukte dieser Katalysatoren, wie bei allen anderen Systemen auch, sind Methacrylsäure sowie andere Nebenprodukte.

In EP 1 393 800 werden Gold-haltige Katalysatoren beschrieben, wobei die als aktive Oxidationsspezies beschriebenen katalytischen Goldpartikel insbesondere einen durchschnittlichen Durchmesser von weniger als 6 nm aufweisen müssen. Besagte Goldpartikel befinden sich verteilt auf einem Siliziumoxid- bzw. einem TiO₂/SiO₂-Träger. Als zusätzliche Aktivkomponenten neben dem Gold, enthalten solche Katalysatoren unter anderem auch andere Metalle in oxidischer Form. Diesen Dotierungskomponenten wird ein synergistischer und Aktivitäts- und Selektivitäts-steigernder Effekt zugeschrieben.
Die Herstellung erfolgt durch Auftragen des Goldsalzes und weiteren Metallsalzen auf einen oxidischen Träger.

Haruta et al beschreiben in J. Catal. 1993, Vol. 144, pp 175-192, dass Goldnanopartikel aufgetragen auf Übergangsmetalloxidischen Trägern, wie TiO₂, Fe₂O₃ oder Co₃O₄ aktive Oxidationskatalysatoren darstellen. Dabei spielt eine Wechselwirkung zwischen Gold und Übergangsmetall eine entscheidende Rolle für die Katalysatoraktivität.

In EP 2 177 267 und EP 2 210 664 werden nickelhaltige Katalysatoren mit Schalenstruktur beschrieben. Selektivität zu MMA beträgt bei diesen Katalysatoren bis zu 97%. Die Raum-Zeit-Ausbeute wird mit 9,7 mol MMA/(kg h) bei einem Goldanteil im Katalysator von ca. 1 Gew% beschrieben. Ein NiOₓ/Au-Katalysator zeigt gemäß Beispielen deutlich bessere Aktivitäten und Selektivitäten zu MMA, während andere Kombinationen, wie z.B. Au mit CuO oder auch Co₃O₄ viel weniger aktiv und selektiv sind. Grundsätzlich ist dies eine Weiterentwicklung der zuvor beschriebenen Katalysatoren, wobei die inhomogene Verteilung des Gold-Ni-Oxid Composite-Partikels sowie eine nicht aktive Katalysatorschale diese neuen Katalysatoren charakterisieren. Auch in diesen wird auf die Bildung von Methacrylsäure als Nebenprodukt verwiesen.

In EP 2 210 664 wird ein Katalysator offenbart, der im Außenbereich in Form einer so genannten Egg-Shell-Struktur, Nickeloxid und Goldnanopartikel auf einem Träger aus SiO₂, Al₂O₃ und einem basischen Element, insbesondere einem Alkali- oder Erdalkalimetall, aufweist. Das Nickeloxid ist dabei an der Oberfläche angereichert, jedoch auch in tieferen Schichten des Katalysatorpartikels in geringeren Konzentrationen enthalten.

In WO 2017/084969 A1 werden Katalysatorsysteme auf Basis von mehreren Mischoxiden als Träger beschrieben, die ebenfalls nanopartikuläres Gold neben Kobaltoxid als Aktivkomponente aufweisen, Die Verteilung der katalytisch aktiven Komponenten, namentlich Gold und Kobalt sind anisotrop über den Querschnitt des Korns verteilt. Weitere neuere Katalysatoren für besagte Reaktion sind in US 9,676,699 beschrieben. Hier werden ähnliche Trägersysteme auf Basis von Silika-, Alumina-, Erdalkalioxid Mischungen, die Palladium neben Bismut mit verschiedenen Drittdotierungen aufweisen, beschrieben. Auch hier wird auf den Zusammenhang zwischen der Wasserkonzentration und der sich als Nebenprodukt bildenden Methacrylsäure hingewiesen.

So unterschiedlich diese Katalysatorsysteme bezüglich der verwendeten Trägermaterialen sind, so unterschiedlich sind auch deren Präparation und letztlich auch die Performance bezüglich Umsatz und Selektivität. Dennoch führen alle diese Katalysatorsysteme zu ähnlichen Nebenproduktcharakteristika. Allen Katalysatoren ist gemein, dass sich im stationären Betrieb neben dem gewünschten Alkylmethacrylat, auch mehr oder weniger große Mengen an Methacrylsäure, Alkoxyisobuttersäureester (AIBSM), sowie Dimere des eingesetzten Methacroleins und Alkylester dieser Dimere bilden. Daneben bilden sich weitere Nebenprodukte wie Hydroxyisobuttersäure und deren korrespondierenden Ester. Relevant sind diese Nebenprodukte vor allem, da sie gegenüber dem gewünschten Alkylmethacrylat hochsiedend sind, und sich bei der Aufarbeitung gegenüber MMA in schwersiedenden Fraktionen anreichern und letztlich sammeln.

### Bezugsverzeichnis zu Figur 1

Figur 1 bildet beispielhaft ein mögliches Fließbild des erfindungsgemäßen MMA-Verfahrens inklusive Reaktor zur Gewinnung von MMA aus MIBSM und MAS dar.
(1) Reaktor I zur MAL-Synthese
(2) Destillationskolonne
(3) Reaktor II zur DOE Reaktion
(4) MAL Abtrennung
(5) Zwischenkolonne und/oder Extraktion
(6) Kolonne zur Methanol-Abtrennung
(7) Kolonne zur MMA-Aufreinigung - Schwersieder
(8) 2. Kolonne zur MMA-Aufreinigung - Leichtsieder
(9) 3. Kolonne zur MMA-Aufreinigung - Reinkolonne
(10) Gereinigtes MMA
(11) Optionale Kolonne zur Reduzierung der MMA Menge aus Sumpfstrom von (7)
(12) Zugabe Säure und MeOH für (13)
(13) Reaktor III zur Spaltung von MIBSM in MMA und DIMAL-Ester in MAL und MMA sowie Veresterung von MAS zu MMA
(14) Optionale Kolonne zur Trennung der Wertstoffe von Schwersiedern & Schwefelsäure
(15) Abwasser
(16) Rückführung für Methacrolein und Methanol

### Experimenteller Teil:

### Beispiel 1: Durchführung der Reaktion bei Normaldruck als Feed-Batch mit frischen Edukten, MAS und MIBSM.

Die Reaktion wird in einem Dreihalskolben aus Glas mit aufgesetzter Kolonne durchgeführt.

Der Dreihalskolben ist mit einem KPG Rührer ausgestattet, sowie einer 1 m hohen Kolonne mit einem freien Durchmesser von 40 mm, die Beheizung erfolgt über ein Ölbad. Die Kolonne ist mit Raschigringen gefüllt, am Kopf des Kolonnenschusses ist ein Rücklaufteiler aufgesetzt, um Rücklauf und Abnahme kontrollieren zu können. In einem 1 I Dreihalskolben werden 2 Mol MIBSM und 2 Mol Methacrylsäure vorgelegt, sowie 0,2 Mol Wasser.

Zu dieser Mischung werden jeweils 200 ppm Phenothiazin sowie 50 ppm Tempol als Stabilisatoren bzw. zur Inhibierung einer radikalischen Polymerisation von (meth)acrylischen Edukten und Produkten unter den Reaktionsbedingungen gegeben. Die Reaktionsmischung wird mittels Ölbad auf 150°C erwärmt, nach 10 min ist diese Temperatur bei vorgeheiztem Ölbad erreicht, die Kolonne auf vollen Rücklauf gestellt, so dass zunächst kein Destillat anfällt. Nach Erreichen der Innentemperatur von 150°C wird kontinuierlich eine Mischung aus MIBSM, MAS und MeOH, Wasser und Schwefelsäure mit einer Dosiergeschwindigkeit von 150 g/h über die abgetauchte Kapillare in das Reaktionsgemisch zugeführt. Die Dosierung der Feedmischung erfolgt über eine HPLC Pumpe, eine zweite HPLC Pumpe führt über eine Kapillare nachdem die Reaktion stationär eingefahren ist, den entstehenden Reaktionssumpf ab.

Edukte sowie Katalysator, Alkohol und Wasser werden separat vorgemischt und über eine Kapillare, die bis unter den Rührer geführt sind in die Reaktion eingebracht. Zusammensetzung der Feedmischung:

**Tabelle 1: Feed Mischung zur Spaltung von MIBSM zu MMA bei paralleler Veresterung von MAS zu MMA.**

| Chemikalie | Gew% | M [g/mol] | Feed [g/h] | Feed [mol/h] | Mol% [Basis = MIBSM) |
|---|---|---|---|---|---|
| MIBSM | 44 | 118 | 66 | 0,56 | 100 |
| MAS | 32 | 86 | 48 | 0,56 | 100 |
| Wasser | 3,3 | 18 | 4,95 | 0,28 | 49 |
| Schwefelsäure | 3,7 | 98 | 5,55 | 0,06 | 10 |
| Methanol | 17 | 32 | 25,5 | 0,80 | 142 |

Somit entspricht das molare Verhältnis der C4-Nebenprodukte MAS und MIBSM 1:1. Der Wassergehalt beträgt 49 mol% bezüglich MIBSM und 142mol% MeOH bezgl. MIBSM.

Das Ölbad wird mit beginnender Feeddosierung auf 160°C hochgeheizt, die Innentemperatur im Reaktor steigt allmählich bis etwa 150°C und am Kopf der Kolonne sammelt sich ein Gemisch bestehend aus den azeotropen Zusammensetzungen der binären Azeotrope MeOH und MMA bzw. MMA und Wasser. Sobald der Kopf der Kolonne eine stabile Temperatur von 69°C erreicht hat wird ein Rücklaufverhältnis von 0,8 eingestellt und Destillat abgenommen.

Die Reaktion wird kontinuierlich zunächst 6 h betrieben, wobei jeweils jede Stunde die Destillatmenge quantifiziert und analysiert wurde. Die Anlage wird so betrieben dass im Mittel pro Stunde etwa 90% der zugeführten Eduktmasse am Kopf als Destillat abgezogen werden, während der Reaktionssumpf ebenfalls kontinuierlich ausgeschleust wird, mittels HPLC Pumpe werden durchschnittlich etwa 10% des zugeführten Feedstroms entfernt. Im Mittel bleibt somit der Füllstand im Kolben erhalten und die Reaktion ist in diesem Stadium als Volumen- bzw. Masse-stationär zu betrachten. Am Kopf der Kolonne nachgeschaltet dem Kondensator, der mit Leitungswasser mit einer Kühlwassertemperatur von etwa 18°C betrieben wird, wird eine Kühlfalle installiert, um leichtflüchtige Komponenten zu erfassen; die Kühlfalle wird mit einer Mischung aus Aceton/Trockeneis betrieben bei knapp minus 60°C, die Kühlfalle ist mit THF gefüllt um leichtflüchtige Komponenten zu absorbieren und qualitativ aufzuklären und zu bestimmen.

Der Sumpf verfärbt sich zunächst gelblich, innerhalb von 6 h dann hellorange, ein Anstieg der Viskosität ist kaum zu bemerken.

Das stationär als Destillat erhaltene Kopfprodukt der Kolonne wiegt 134,9 g/h und setzt sich laut GC Chromatographie wie folgt zusammen:

**Tabelle 2: Destillatprodukt der Reaktion.**

| Chemikalie | Gew% | M [g/mol] | Destilat [g/h] | Destilat [mol/h] |
|---|---|---|---|---|
| MMA | 79,1 | 100 | 106,7 | 1,06 |
| Wasser | 3,3 | 18 | 4,7 | 0,26 |
| Methanol | 17,6 | 32 | 23,7 | 0,74 |
| | | | | |
| Destillat Gesamt | 100 | # | 134,9 | 2,06 |

Das stationär erhaltene Sumpfprodukt der Ausschleusung wiegt 15,1 g/h und setzt sich wie folgt zusammen:

**Tabelle 3: Sumpfprodukt der Reaktion.**

| Chemikalie | Gew% | M [g/mol] | Sumpf [g/h] | Sumpf [mmol/h] |
|---|---|---|---|---|
| MIBSM | 17,5 | 118 | 2,64 | 22,4 |
| MAS | 21,9 | 86 | 3,30 | 38,4 |
| Methanol | 0,1 | 32 | 0,01 | 0,3 |
| Schwefelsäure | 36,8 | 98 | 5,55 | 56,6 |
| Wasser | 1,7 | 18 | 0,25 | 13,9 |
| Hochsieder | 22,2 | # | 3,35 | # |
| | | | | |
| Sumpf Gesamt | 100 | # | 15,1 | # |

Das entspricht bezüglich der zugeführten Eduktmengen einer rechnerischen Ausbeute an MMA bezgl. MIBSM von 96%, bezüglich der zu MMA veresterten Methacrylsäure von 95% und einer Methanol Wiedergewinnungsrate von 93%.

In der Kühlfalle werden neben dem als Absorptionsmittel THF eingesetzten Lösungsmittel geringe Mengen an Dimethylether mittels Gaschromatographie (Siedepunkt -24°C) detektiert.

Der Versuch zeigt, dass bei den gewählten Bedingungen aus Mischungen enthaltend MAS und MIBSM in Gegenwart stöchiometrischer Mengen an Schwefelsäure und in Gegenwart von MeOH und Wasser hocheffizient und bei hohen Umsetzungsraten (bzgl. der Edukte) Roh-MMA hergestellt werden kann.

### Beispiel 2 bis 9:

Herstellung von Methacrolein aus Propanal und Formalin: Methacrolein wurde gemäß EP 2 998 284 hergestellt und isoliert.

Eine Formalinlösung mit einem Formalingehalt je nach Beispiel von 37 Gew% oder 55 Gew% und Propanal werden mittels eines statischen Mischers vermischt (im Weiteren als Aldehydlösung bezeichnet) und anschließend in einem mit Öl beheizten Wärmetauscher auf die gewünschte Temperatur (siehe Tab.1) erwärmt. Der genaue Wassergehalt des Formalins, abhängig vom Beispiel, spielt keine weitere Rolle, da dieser in den Wassergehalt des Frisch-Feeds gemäß Tabelle 1 vollständig mit eingeht. Ein Recyclestrom aus dem Sumpf der Produktkolonne, die an den Rohrreaktor anschließt, wird mit Essigsäure und Dimethylamin (als 40 %ige Lösung in Wasser) vermischt und ebenfalls auf die gewünschte Temperatur vorgewärmt. Die vorgewärmte Aldehydlösung und die vorgewärmte Katalysatorlösung werden in einem weiteren statischen Mischer vermischt. Diese Eduktmischung wird dann einem mittels Öl temperierten Rohrreaktor zugeführt. Üblicherweise wird die Reaktion bei Drücken von ca. 35 bis 40 bar durchgeführt.

Die Produktmischung am Ablauf des Rohrreaktors wird über ein Ventil entspannt und gelangt in die Produktkolonne zur Destillation. Am Kopf dieser Kolonne wird nach Kondensation und Phasentrennung ein zweiphasiges Gemisch aus Methacrolein und einer wässrigen Phase erhalten. Die wässrige Phase wird in die Kolonne zurückgeführt. Die organische Phase gelangt in die Produktvorlage. Am Sumpf der Kolonne wird ein Teilstrom als Recycle in die Reaktion zurückgeführt. Ein weiterer Teilstrom wird als wässriges Produkt in eine weitere Produktvorlage abgeführt. In Beispiel 1 bis 4 wird eine Methacrolein Qualität mit einem DIMAL-Gehalt kleiner 0,2 Gew% erhalten. Der Wassergehalt beträgt ca. 56 Gew% und der auf das Wasser im Feed bezogene Dimethylamin-Gehalt beträgt ca. 2,7 Gew%. Die Temperatur im Reaktor liegt zwischen 122°C als Eingangstemperatur und 153°C als Austrittstemperatur. Eine erhebliche Temperaturspitze tritt nicht auf.

Beispiele 5 bis 7 zeigen, dass die Parameter der Reaktionsführung maßgeblich Umsatz und DIMAL Gehalt beeinflussen, da hier zwar ein Gehalt an dimeren MAL unter 0,4 Gew%, nicht jedoch unterhalb von 0,2 Gew% realisiert werden konnte. Der Unterschied zu den Beispielen 1 bis 4 ist hier neben einer teilweise höheren Eingangstemperatur vor allem die höheren Maximaltemperatur und Ausgangstemperatur.

Beispiele 8 und 9 zeigen Ausführungsformen die eine Methacrolein Qualität erzeugen mit einem Gehalt an dimeren MAL unter 0,5 Gew%. Hier waren die Eingangstemperaturen und insbesondere die Maximaltemperaturen noch höher. Insbesondere lagen die Maximaltemperaturen oberhalb der bevorzugten Maximaltemperaturen von 165 °C oder sogar von 170°C.

**Tabelle 4: Herstellung von MAL aus Propionaldehyd und Formalin.**

| **Tabelle: Herstellung von Methacrolein aus Propanal (PA) und Formalin (FO)** | PA:FO | DMA:PA | ACOH:DMA | Recycle | DMA:PA | H₂O | DMA/H2O | VWZ | T_{ÖL} | Tₑᵢₙ | Tₘₐₓ | Tₐᵤₛ | Umsatz PA | Selektivität MAL | c DIMAL |
|---|---|---|---|---|---|---|---|---|---|---|---|---|---|---|---|
| | Frischfeed | | | | Reaktoreingang | | | | | | | | | | |
| | mol/mol | mol% | mol/mol | % | mol% | % | % | sec | °C | °C | | °C | % | % | % |
| DE3213681A1 Bsp1 | 1 | 3,7 | 1,08 | - | - | 50 | 1,8 | 6,9 | | 161 | 184 | - | 99,5 | 98,1 | 0,49 |
| DE3213681A1 Bsp2 | 1 | 3,6 | 1,14 | - | - | 40 | 2,5 | 6 | | 162 | 205 | - | > 99,4 | 97,2 | <1 |
| Beispiel 1 | 0,99 | 2,50 | 1,09 | 70,5 | 7,8 | 55,6 | 2,74 | 9,30 | 139,5 | 122,5 | 152,6 | 152,2 | 99,37 | 98,75 | 0,18 |
| Beispiel 2 | 0,99 | 2,51 | 1,09 | 71,0 | 7,8 | 56,1 | 2,74 | 9,26 | 139,1 | 122,5 | 152,3 | 152,0 | 99,30 | 98,85 | 0,18 |
| Beispiel 3 | 0,98 | 2,61 | 1,09 | 71,2 | 8,2 | 54,9 | 2,82 | 9,41 | 139,9 | 122,1 | 152,3 | 152,2 | 99,35 | 98,67 | 0,18 |
| Beispiel 4 | 0,96 | 2,51 | 1,09 | 70,1 | 7,7 | 56,5 | 2,71 | 9,21 | 139,1 | 122,8 | 153,0 | 153,0 | 99,46 | 98,33 | 0,18 |
| Beispiel 5 | 0,99 | 2,51 | 1,09 | 70,5 | 7,8 | 55,7 | 2,75 | 9,26 | 143,9 | 129,9 | 160,2 | 155,5 | 99,75 | 98,19 | 0,34 |
| Beispiel 6 | 0,99 | 2,51 | 1,09 | 70,4 | 7,8 | 55,6 | 2,75 | 9,30 | 144,2 | 127,3 | 157,7 | 154,7 | 99,65 | 98,47 | 0,27 |
| Beispiel 7 | 0,98 | 2,50 | 1,09 | 70,5 | 7,7 | 56,0 | 2,72 | 9,22 | 139,0 | 122,5 | 156,3 | 154,9 | 99,57 | 98,62 | 0,22 |
| Beispiel 8 | 0,99 | 2,51 | 1,09 | 70,5 | 7,8 | 55,6 | 2,74 | 9,26 | 159,8 | 142,1 | 173,0 | 169,1 | 99,67 | 98,03 | 0,49 |
| Beispiel 9 | 0,99 | 2,52 | 1,08 | 70,4 | 7,8 | 55,6 | 2,76 | 9,26 | 146,4 | 133,8 | 165,4 | 159,7 | 99,77 | 98,34 | 0,45 |

Das wie oben beschrieben hergestellte Methacrolein wird nach der Reaktion entspannt (optional in einer Flashbox teilweise verdampft), und in eine Destillationskolonne geführt. Am Kopf der Destillationskolonne erhält man nach Kondensation ein zweiphasiges Gemisch (je nach Temperatur trennt sich eine mehr oder weniger große Wasserphase ab), wobei die obere Phase Methacrolein in einer Qualität von > 97% enthält, mit einem Wassergehalt von 1-3 wt%. Der Formalingehalt im Methacrolein beträgt < 2000 ppm, der Methanolgehalt beträgt in Abhängigkeit des Methanolgehalts des eingesetzten Formalin zwischen 0,1 bis 1,0 wt%. Gemäß obigen Beispielen enthält das Methacrolein einen DiMAL Gehalt von 0,18 wt% bis < 1wt%. Diese Qualität wird in den folgenden Versuchen für die direkte oxidative Veresterung mit Methanol eingesetzt.

### Beispiel 10: Durchführung der direkten oxidativen Veresterung in der Flüssigphase

Ein 20 L Reaktor mit Begasungsrührer wird mit einem Reaktionsgemisch aus 36 Gewichtsprozent Methacrolein in Methanol befüllt bei einer Slurrydichte von 8 Gewichtsprozent Katalysator. Das Reaktionsgemisch wird unter Rühren bei 80 °C auf 5 bar gebracht und so Luft zu dosiert, dass die Sauerstoffkonzentration im Restgas nach den Kondensatoren 4.0 Volumenprozent beträgt. Der pH-Wert wird durch kontinuierliche Einbringung von 4 gewichtsprozentiger NaOH in Methanol Lösung auf 7 eingestellt. Das Reaktionsgemisch wird kontinuierlich aus dem Reaktor abgeführt in der Form das die Katalysatorbelastung bei 11 mol MAL / kg Katalysator / Stunde liegt. Die Laufzeit beträgt jeweils 1000 Stunden.
a) Als Katalysator wird ein Gold-Cobaltoxid Katalysator (WO2017084969 A1) eingesetzt und ein Umsatz von 76% MAL bei einer Selektivität von 94,1% MMA erhalten wird. Die Selektivität zu MAS beträgt 3,1 % und die Selektivität zu MIBSM 1.2%
b) Als Katalysator wird ein Gold-Nickeloxid Katalysator (US8450235) eingesetzt und ein Umsatz von 75% MAL bei einer Selektivität von 94,4% MMA erhalten wird. Die Selektivität zu MAS beträgt 2,5% und die Selektivität zu MIBSM 1.2%
c) Als Katalysator wird ein Palladium-Blei Katalysator (US5969178) eingesetzt, wobei der pH-Wert der Reaktion auf 6.3 eingestellt wird ist und ein Umsatz von 60% MAL bei einer Selektivität von 89% MMA erhalten wird. Die Selektivität zu MAS beträgt 7% und die Selektivität zu MIBSM liegt unter 0,1%.
d) Als Katalysator wird ein Palladium-Bismuth-Tellurium Katalysator (US20160168072) eingesetzt und die Bedingungen und Stöchiometrien werden gemäß Beispiel 2 und 3 wie in US20160168072 beschrieben, eingestellt. Man erhält einen Umsatz von 89% bei einer Selektivität von 92% MMA. Die Selektivität zu MAS liegt unterhalb von 0.2% und die Selektivität zu MIBSM liegt bei 1.2%.

### Beispiel 11: Spaltung von MIBSM zu MMA und Methanol bei gleichzeitiger Veresterung von MAS zu MMA mit Reaktionsmischungen aus Beispiel 3 nach erfindungsgemäßer Abtrennung von Methacrolein und Methylmethacrylat. Schwefelsäure als Katalysator

Es wurde das aus Beispiel 3a erhaltende Reaktion Gemisch nach der Aufarbeitung genommen: Die Aufarbeitung ist exemplarisch beschrieben, wobei die jeweiligen Zusammensetzungen in Tabelle 1 aufgeführt sind.

Der Austrag von Reaktor II (1000g/hr) wurde auf die MAL-Recovery Kolonne auf Stufe 11 von 22 geleitet. Die Temperatur im Sumpf war 70 °C bei einem Druck von 930 mbar. Der Sumpfstrom wurde mit Schwefelsäure auf pH 2 angesäuert, in einem Dekanter getrennt und die Organik in den Sumpf der Extraktionskolonne gefahren, wobei die wässrige Phase in den Kopf der 30-Stufigen Extraktionskolonne eingeleitet wurde. Die Sumpftemperatur der Extraktionskolonne lag bei 43,9 °C bei einem Druck von 1013 mbar. Der Kopfstrom der Extraktionskolonne wurde auf die Stufe 6 von 10 der Schwersieder Kolonne eingeleitet. Die Sumpftemperatur betrug 85,4 °C bei einem Druck von 235 mbar.

**Tabelle 5: Zusammensetzung des Reaktionsgemisches bei den verschiedenen Aufarbeitungs-schritten.**

| Komponente | Reaktoraustrag | Sumpf MAL-Recovery | Kopf Extraktion | Sumpf Schwersieder |
|---|---|---|---|---|
| Methanol | 47,3% | 28,9% | 1,8% | 22 ppm |
| Wasser | 5,6% | 10,9% | 6,0% | 0,3% |
| MAL | 10,3% | 64 ppm | 1300 ppm | 12 ppm |
| MMA | 32,6% | 55,1% | 83,4% | 11,4% |
| MIBSM | 0,4% | 1,2% | 1,4% | 32,2% |
| MAS | 0,9% | 0,6% | 2,8% | 24,6% |
| DIMAL-Ester | 0,1 | 0,3% | 0,7% | 12,8% |
| Nebenkomponenten | 2,8% | 2,1% | 3,8% | 19,0% |

Der Sumpf der Schwersieder Kolonne wurde gesammelt und für die Spaltung von MIBSM in MMA und MeOH sowie DIMAL-Ester in MAL und MMA bei gleichzeitiger Veresterung von MAS mit MeOH zu MMA eingesetzt.

Ein 500 mL Dreihalskolben wurde mit einer Kolonne und einem Glasthermometer versehen. Am Kopf der Kolonne wurden 50 g MeOH mit Phenothiazin (ca. 500 ppm) in einem Tropftrichter angebracht, um durch kontinuierliche Zugabe eine Polymerisation in der Kolonne zu verhindern. Das Thermoelement wurde im Ölbad (T(ÖI)=165°C) platziert.

300 g Feed (1 Eq., 0,73 mol 3-MibsM; 1,17 Eq., 0.85 mol MAS; 0,34 mol MMA, 0,23 mol DIMAL-Ester),
2,84 g (0,04 Eq., 0,029 mol) H₂SO₄ und
13,42 g H2O (1,02 Eq., 0,75 mol)
wurden in dem Dreihalskolben vorgelegt und in das Ölbad (Soll Temperatur = 165 °C) geführt.

Das Gemisch wurde 3 h auf 165°C (Ölbad-Soll) erhitzt, wobei eine Sumpftemperatur von 151 °C erreicht wurde. Die Destillatabnahme erfolgte kontinuierlich und wurde per HPLC analysiert. Über den Verlauf der Reaktion von 3 Stunden wurde die Methanol-Stabilisator Lösung (6,54 g/h, 0,20 mol) zugegeben

Die folgende Tabelle Nr.2 zeigt die eingesetzten Mengen an Schwefelsäure, Wasser, Methanol und Feed Probe. Auch die Zusammensetzung der Feed Probe für 3-MibsM, MAS und MMA samt der molaren Massen ist aufgeführt.

**Tabelle 6: Menge der eingesetzten Stoffe und deren molare Massen.**

| | **Feed [Gew.-%]** | **H₂SO₄ [Gew.-%]** | **H₂O [Gew.-%]** | **MeOH [Gew.-%]** | **Stabi [Gew.-%]** | **Gesamt [g]** | **Mol. Masse** |
|---|---|---|---|---|---|---|---|
| **3-MibsM** | 32,31 | | | | | | 132,16 |
| **MAS** | 24,61 | | | | | | 86,09 |
| **MMA** | 11,41 | | | | | | 100,12 |
| **DIMAL-Ester** | 12,8 | | | | | | 170,21 |
| **H2SO4** | | 98,00 | | | | | 98,08 |
| **H2O** | | 2,00 | 100,00 | | | | 18,02 |
| **MeOH** | | | | 100,00 | | | 32,04 |
| **S47** | | | | | | | |
| **S71** | | | | | | | |
| **Massen [g]** | **300,51** | **2,84** | **13,42** | **19,63** | **0,26** | **362,57** | |

Tabelle Nr.7 zeigt die Wiederfindung der eingesetzten Massen der oben aufgeführten Edukte.

**Tabelle 7: Schließung der Massenbilanz**

| **Eingesetzte Menge [g]** | **Destillat [g]** | **Sumpf [g]** | **Dest+Sumpf [g]** | **Differenz [g]** | **Bilanzierung** |
|---|---|---|---|---|---|
| 362,57 | 232,81 | 125,78 | 358,59 | 3,98 | 98,90% |

Die Massenbilanz liegt bei einer Wiederfindung von 98,90%. Tabelle Nr.4 zeigt die molare Verteilung der Komponenten in Destillat und Sumpf.

**Tabelle 5: Verteilung der Komponenten in Destillat und Sumpf**

| **Komp.** | **Vorlage [mol]** | **Dest. [mol]** | **Sumpf [mol]** | **Delta Mol** | **Umsatz/Sel.** | |
|---|---|---|---|---|---|---|
| **3-MIBSM** | 0,735 | 0,000 | 0,029 | 0,705 | **96,00%** | Umsatz an 3-MIBSM |
| **MAS** | 0,859 | 0,001 | 0,108 | 0,750 | **87,30%** | Umsatz an MAS |
| **MMA** | 0,342 | 1,620 | 0,166 | 1,443 | **99,16%** | Selektivität/(MAS+3-MIBSM) |
| **MAL** | 0 | 0,019 | 0 | 0,019 | **8,26%** | Umsatz DIMAL-Ester zu MAL und MMA |
| **MeOH(*)** | 0,613 | 0,23 | | - | **-** | |
| **H2O** | 0,753 | | | - | - | |

Der Versuch war erfolgreich, es kam zu einer hohen Umsetzung von 3-MIBSM und MAS zu MMA.

Es ergab sich eine Umsetzung von 96,0% 3-MIBSM zu MMA und 87,3% Umsetzung von MAS zu MMA. Die Selektivität zu MMA betrug für MAS und 3-MIBSM in Summe 99,16%. Der Umsatz der thermischen Spaltung von DIMAL-Ester in MAL und MMA betrug 8,26%.

### Beispiel 12: Spaltung von MIBSM zu MMA und Methanol bei gleichzeitiger Veresterung von MAS zu MMA mit Reaktionsmischungen aus Beispiel 3 nach erfindungsgemäßer Abtrennung von Methacrolein und Methylmethacrylat. Phosphorsäure als Katalysator

Die Reaktion wurde analog zu Beispiel 11 durchgeführt und Phosphorsäure anstelle von Schwefelsäure eingesetzt.

Der Versuch war erfolgreich, es kam zu einer hohen Umsetzung von 3-MIBSM und MAS zu MMA. Es ergab sich eine Umsetzung von 94,2% 3-MIBSM zu MMA und 86,8% Umsetzung von MAS zu MMA. Die Selektivität zu MMA betrug für MAS und 3-MIBSM in Summe 99,0%. Der Umsatz der thermischen Spaltung von DIMAL-Ester in MAL und MMA betrug 8,25%.

### Beispiel 13: Spaltung von MIBSM zu MMA und Methanol bei gleichzeitiger Veresterung von MAS zu MMA mit Reaktionsmischungen aus Beispiel 3 nach erfindungsgemäßer Abtrennung von Methacrolein und Methylmethacrylat. Methansulfonsäure als Katalysator

Die Reaktion wurde analog zu Beispiel 11 durchgeführt und Methansulfonsäure anstelle von Schwefelsäure eingesetzt.

Der Versuch war erfolgreich, es kam zu einer hohen Umsetzung von 3-MIBSM und MAS zu MMA. Es ergab sich eine Umsetzung von 92,6% 3-MIBSM zu MMA und 84,7% Umsetzung von MAS zu MMA. Die Selektivität zu MMA betrug für MAS und 3-MIBSM in Summe 98,8%. Der Umsatz der thermischen Spaltung von DIMAL-Ester in MAL und MMA betrug 8,20%.

### Beispiel 14: Spaltung von MIBSM zu MMA und Methanol bei gleichzeitiger Veresterung von MAS zu MMA mit Reaktionsmischungen aus Beispiel 3 nach erfindungsgemäßer Abtrennung von Methacrolein und Methylmethacrylat. Temperatur von 120 °C

Die Reaktion wurde analog zu Beispiel 11 durchgeführt bei einer Temperatur von 120 °C.

Es kam zu einer hohen Umsetzung von 3-MIBSM und MAS zu MMA.

Es ergab sich eine Umsetzung von 60,4% 3-MIBSM zu MMA und 87,3% Umsetzung von MAS zu MMA. Der Umsatz der thermischen Spaltung von DIMAL-Ester in MAL und MMA betrug 8,20%.

### Beispiel 15: Spaltung von MIBSM zu MMA und Methanol bei gleichzeitiger Veresterung von MAS zu MMA mit Reaktionsmischungen aus Beispiel 3 nach erfindungsgemäßer Abtrennung von Methacrolein und Methylmethacrylat. Temperatur von 90 °C

Die Reaktion wurde analog zu Beispiel 11 durchgeführt bei einer Temperatur von 90 °C.

Es kam zu keiner hohen Umsetzung von 3-MIBSM zu MMA; aber von MAS zu MMA.

Es ergab sich eine Umsetzung von kleiner 1% 3-MIBSM zu MMA und 88,0% Umsetzung von MAS zu MMA. Die thermische Spaltung von DIMAL-Ester in MAL und MMA lief nicht ab.

### Beispiel 16: Spaltung von MIBSM zu MMA und Methanol bei gleichzeitiger Veresterung von MAS zu MMA mit Reaktionsmischungen aus Beispiel 3 nach erfindungsgemäßer Abtrennung von Methacrolein und Methylmethacrylat. Erhöhte Menge an Schwefelsäure

Die Reaktion wurde analog zu Beispiel 11 durchgeführt mit einer erhöhten Schwefelsäuremenge von 40 mol%.

Der Versuch war erfolgreich, es kam zu einer hohen Umsetzung von 3-MIBSM und MAS zu MMA. Es ergab sich eine Umsetzung von 95,9% 3-MIBSM zu MMA und 87,4% Umsetzung von MAS zu MMA. Die Selektivität zu MMA betrug für MAS und 3-MIBSM in Summe 99,14%. Der Umsatz der thermischen Spaltung von DIMAL-Ester in MAL und MMA betrug 8,16%.

### Vergleichsbeispiel. 1: Spaltung von MIBSM zu MMA und Methanol bei gleichzeitiger Veresterung von MAS zu MMA mit Reaktionsmischungen aus Beispiel 3 nach erfindungsgemäßer Abtrennung von Methacrolein und Methylmethacrylat. Temperatur von 23 °C

Die Reaktion wurde analog zu Beispiel 11 durchgeführt bei einer Temperatur von 23 °C.

Es kam zu keinen hohen Umsetzungen von 3-MIBSM zu MMA und von MAS zu MMA.

Es ergab sich eine Umsetzung von kleiner 1% 3-MIBSM zu MMA und 20% Umsetzung von MAS zu MMA. Die thermische Spaltung von DIMAL-Ester in MAL und MMA lief nicht ab.

### Vergleichsbeispiel. 2: Spaltung von MIBSM zu MMA und Methanol bei gleichzeitiger Veresterung von MAS zu MMA mit Reaktionsmischungen aus Beispiel 3 nach erfindungsgemäßer Abtrennung von Methacrolein und Methylmethacrylat. Keine Zugabe von Schwefelsäure als Katalysator

Die Reaktion wurde analog zu Beispiel 11 ohne Zugabe von Schwefelsäure als Katalysator

Es kam zu einer hohen Umsetzung von 3-MIBSM zu MMA aber keine Umsetzung von MAS zu MMA. Es ergab sich eine Umsetzung von 94% 3-MIBSM zu MMA und keiner Umsetzung von MAS zu MMA. Die thermische Spaltung von DIMAL-Ester in MAL und MMA betrug 8,0%.

### Beispiel 17: Kontinuierliche Spaltung von MIBSM zu MMA und Methanol bei gleichzeitiger Veresterung von MAS zu MMA mit Reaktionsmischungen aus Beispiel 3 nach erfindungsgemäßer Abtrennung von Methacrolein und Methylmethacrylat.

Die Reaktion wurde analog zu Beispiel 11 durchgeführt. Zusätzlich wurde nach Erreichen der Sumpftemperatur von 151 °C mit einem kontinuierlichen Zulauf an Feed Mischung von 57 g/hr begonnen. Der Versuch lief für 6 Stunden.

Der Versuch war erfolgreich, es kam zu einer hohen Umsetzung von 3-MIBSM und MAS zu MMA. Es ergab sich eine Umsetzung von 95,5% 3-MIBSM zu MMA und 87,5% Umsetzung von MAS zu MMA. Die Selektivität zu MMA betrug für MAS und 3-MIBSM in Summe 98,7%. Der Umsatz der thermischen Spaltung von DIMAL-Ester in MAL und MMA betrug 8,05%.

## Patentansprüche

1. Verfahren zur Herstellung von Alkylmethacrylaten, bei dem in einer ersten Reaktionsstufe in einem Reaktor I Methacrolein hergestellt und dieses in einer zweiten Reaktionsstufe in einem Reaktor II oxidativ mit einem Alkohol zu einem Alkylmethacrylat verestert wird, **dadurch gekennzeichnet, dass**
a. der Reaktoraustrag des Reaktor II in eine den überwiegenden Teil des Alkylmethacrylat enthaltene Fraktion und eine zweite Fraktion, enthaltend Methacrylsäure und einen Alkoxyisobuttersäurealkylester getrennt wird, und dass
b. diese zweite Fraktion in einem Reaktor III derart umgesetzt wird, dass aus dem Alkoxyisobuttersäurealkylester und der Methacrylsäure weiteres Alkylmethacrylat gebildet wird.

2. Verfahren gemäß Anspruch 1, **dadurch gekennzeichnet, dass** in Schritt a die Trennung mittels mindestens einer Extraktion und/oder einer Destillation erfolgt.

3. Verfahren gemäß Anspruch 1 oder 2, **dadurch gekennzeichnet, dass** die Umsetzung gemäß Schritt b. bei einer Temperatur durchgeführt wird, die gleich oder höher ist als die oxidative Umsetzung in Reaktor II,

4. Verfahren gemäß mindestens einem der Ansprüche 1 bis 3, **dadurch gekennzeichnet, dass** die Umsetzung gemäß Schritt b mit einem Reaktionsgemisch durchgeführt wird, das neben Alkoxyisobuttersäurealkylester auch Methacrylsäure, sowie dimeres Methacrolein (DIMAL) und Derivate des dimeren Methacroleins, wie DIMAL-Ester, sowie Wasser und freien Alkohol enthält.

5. Verfahren gemäß mindestens einem der Ansprüche 1 bis 4, **dadurch gekennzeichnet, dass** die zweite Fraktion in Reaktor III bei einer Temperatur von mindestens 90 °C, bevorzugt von mindestens 110 °C umgesetzt wird.

6. Verfahren gemäß mindestens einem der Ansprüche 1 bis 5, **dadurch gekennzeichnet, dass** die zweite Fraktion in Reaktor III gemäß b in Gegenwart eines Katalysators, bevorzugt in Gegenwart einer Brönstedtsäure umgesetzt wird.

7. Verfahren gemäß mindestens einem der Ansprüche 1 bis 6, **dadurch gekennzeichnet, dass** der Reaktoraustrag des Reaktor II in einer ersten Destillationskolonne von Methacrolein und teilweise von dem Alkohol befreit wird, wobei ein Strom, enthaltend Alkylmethacrylat, Wasser, ein Alkalimethacrylat und/oder Methacrylsäure, Alkoxyisobuttersäurealkylester und Alkohol, erhalten wird, der anschließend mit einer starken Säure versetzt und in einer Extraktion in eine hydrophobe Phase, enthaltend Alkylmethacrylat, die größere Fraktion an MAS und Alkoxyisobuttersäurealkylester und eine hydrophile Phase, enthaltend Wasser, den Alkohol, und Anteile Alkylmethacrylat und Methacrylsäure getrennt wird.

8. Verfahren gemäß mindestens einem der Ansprüche 1 bis 6, **dadurch gekennzeichnet, dass** der Reaktoraustrag des Reaktor II in einer ersten Destillationskolonne von Methacrolein und teilweise von dem Alkohol befreit wird, wobei ein Strom, enthaltend Alkylmethacrylat, Wasser, ein Alkalimethacrylat und/oder Methacrylsäure, Alkoxyisobuttersäurealkylester und Alkohol, erhalten wird, der anschließend in einer zweiten Destillation in eine leichte Phase, enthaltend Alkylmethacrylat und den Alkohol und eine schwere Phase, enthaltend Wasser, Alkoxyisobuttersäurealkylester und Methacrylsäure, getrennt wird.

9. Verfahren gemäß mindestens einem der Ansprüche 1 bis 8, **dadurch gekennzeichnet, dass** es sich bei dem Alkohol um Methanol, bei dem Alkylmethacrylat um Methylmethacrylat (MMA) und bei dem Alkoxyisobuttersäurealkylester um Methoxyisobuttersäuremethylester (MIBSM) handelt.

10. Verfahren gemäß mindestens einem der Ansprüche 1 bis 9, **dadurch gekennzeichnet, dass** die Umsetzung in Reaktor III bei einer Temperatur zwischen 80 und 170 °C erfolgt.

11. Verfahren gemäß Anspruch 6, **dadurch gekennzeichnet, dass** es sich bei der Brönstedtsäure in Reaktor III um Schwefelsäure handelt.

12. Verfahren gemäß mindestens einem der Ansprüche 1 bis 11, **dadurch gekennzeichnet, dass** es sich bei der ersten Reaktionsstufe in Reaktor I um eine Umsetzung von Propanal mit Formalin handelt.

13. Verfahren gemäß mindestens einem der Ansprüche 1 bis 11, **dadurch gekennzeichnet, dass** es sich bei der ersten Reaktionsstufe in Reaktor I um eine Umsetzung von Isobuten und/oder *tert*-Butanol mit Luftsauerstoff in Gegenwart eines heterogenen Kontakts bei Temperaturen von 300 bis 500°C unter Entstehung von Methacrolein handelt, das kondensiert und zu einer Reinheit von mindestens 80% aufgearbeitet und flüssig isoliert wird und anschließend der weiteren Umsetzung in Reaktor II der oxidativen Veresterung zugeführt wird.

14. Verfahren gemäß Anspruch 12, **dadurch gekennzeichnet, dass** der Reaktoraustrag des Reaktor II in einer ersten Destillationskolonne von Methacrolein und teilweise von dem Alkohol befreit wird, wobei ein Strom, enthaltend Alkylmethacrylat, Wasser, ein Alkalimethacrylat und/oder Methacrylsäure, Alkoxyisobuttersäurealkylester und Alkohol, erhalten wird, der anschließend in einer zweiten Destillation in eine leichte Phase, enthaltend Alkylmethacrylat und den Alkohol und eine schwere Phase, enthaltend Wasser, Alkoxyisobuttersäurealkylester, Methacrylsäure, dimeres Methacrolein und optional einen Alkylester des dimeren Methacroleins, getrennt wird.

15. Verfahren gemäß Anspruch 6 und 11, **dadurch gekennzeichnet, dass** das dimere Methacrolein in Reaktor III in Methacrolein und der optional vorliegende Alkylester des dimeren Methacroleins in Methacrolein und ein Alkylmethacrylat gespalten werden.

16. Verfahren gemäß Anspruch 14 und 15, **dadurch gekennzeichnet, dass** das Methacrolein von dem Alkylmethacrylat in einer späteren Destillationsstufe getrennt und zurück in Reaktor II geführt wird.

17. Verfahren gemäß Anspruch 16, **dadurch gekennzeichnet, dass** der Reaktoraustrag des Reaktor II in einer ersten Destillationskolonne von Methacrolein und teilweise von dem Alkohol befreit wird, wobei ein Strom, enthaltend Alkylmethacrylat, Wasser, ein Alkalimethacrylat und/oder Methacrylsäure, Alkoxyisobuttersäurealkylester und Alkohol, erhalten wird, der anschließend in einer zweiten Destillation oder eine Extraktion in eine leichte oder hydrophobe Phase, enthaltend Alkylmethacrylat und eine schwere oder hydrophile Phase, enthaltend Wasser, Alkoxyisobuttersäurealkylester, Methacrylsäure und Terephthalsäure, getrennt wird, und dass die Terephthalsäure aus dem Reaktoraustrag aus Reaktor III als schwersiedende Komponente destillativ oder als hydrophile Komponente durch Extraktion entfernt wird.

18. Verfahren gemäß mindestens einem der Ansprüche 1 bis 6, **dadurch gekennzeichnet, dass** die eingesetzte Brönstedtsäure in den Reaktor III oder einen anderen Aufarbeitungsschritt rezykliert wird.
